# EUROPÄISCHE PATENTSCHRIFT

(11) **EP 0 580 079 B1**
(45) Veröffentlichungstag und Bekanntmachung des Hinweises auf die Patenterteilung: **11.12.1996**
(21) Anmeldenummer: 93111376.5
(22) Anmeldetag: 15.07.1993
(51) Int. Cl.: C08F 8/12

(54) **Vernetzte, stickstoffhaltige Vinylcopolymere, Verfahren zu ihrer Herstellung sowie die Verwendung dieser Verbindungen**
Crosslinked, nitrogen-containing vinyl copolymers, process for their manufacture and use of these compounds
Copolymères vinyliques réticulés, contenant de l'azote, leur procédé de préparation et leur application

(30) Priorität: 22.07.1992 DE 4224110
(43) Veröffentlichungstag der Anmeldung: 26.01.1994
(73) Patentinhaber: HOECHST AKTIENGESELLSCHAFT, 65926 Frankfurt am Main (DE)
(72) Erfinder: Ahlers, Michael, Dr., D-55122 Mainz (DE); Glombik, Heiner, Dr., D-65719 Hofheim am Taunus (DE); Müllner, Stefan, Dr., D-65239 Hochheim (DE); Walch, Axel, Dr., D-60487 Frankfurt/Main (DE)

(56) Entgegenhaltungen:
- EP-A- 0 071 050
- EP-A- 0 216 387
- EP-A- 0 219 910
- EP-A- 0 339 371
- DE-A- 4 007 312
- FR-A- 1 587 554
- US-A- 4 238 579
- PATENT ABSTRACTS OF JAPAN vol. 10, no. 208 (C-361)(2264) 22. Juli 1986

## Beschreibung

Vernetzte, stickstoffhaltige Vinylcopolymere, Verfahren zu ihrer Herstellung sowie die Verwendung dieser Verbindungen.

Die Erfindung betrifft vernetzte, stickstoffhaltige Vinylcopolymere und ein Verfahren zu deren Herstellung. Aufgrund ihrer Fähigkeiten, Gallensäuren zu adsorbieren, eignen sie sich als Arzneimittel insbesondere zur Senkung des Cholesterinblutspiegels. Sie eignen sich ferner für die Verwendung als Wirkstoffträger, als Nahrungsmittelhilfsstoff und Nahrungsmittelzusatzstoff. Die Erfindung betrifft daher auch die Verwendung der Verbindungen sowie ein Verfahren zu ihrer Herstellung.

Gallensäuren haben eine wichtige physiologische Funktion bei der Fettverdauung. Als Endprodukte des Cholesterinstoffwechsels werden sie in der Leber synthetisiert, in der Gallenblase gespeichert und in den Darm abgegeben, wo sie ihre physiologische Wirkung entfalten. Der größte Teil der sezernierten Gallensäuren wird über den enterohepatischen Kreislauf wieder zurückgewonnen (ca. 20 - 25 g/Tag). Eine Unterbindung dieser Rückresorption vermindert den Gallensäurepool in der Leber und bewirkt so neben einer Stimulierung der Cholesterineigensynthese eine verstärkte Cholesterinaufnahme aus dem Blutkreislauf. Hierzu erhöht sich die Zahl der hepatischen LDL-Rezeptoren auf den Membranen der Leberzellen, so daß die cholesterinhaltigen LDL-Partikel beschleunigt katabolisiert werden und es zu einer Herabsetzung des Cholesterinanteils im Blut kommt.

Es ist bekannt, daß sich Gallensäuren an unlösliche, basische, vernetzte Polymere wie Polyethylenimine (vgl. z. B. EP-A-0379 161) oder Polyvinylimidazole (vgl. EP-B-0162 388) binden lassen und daher als geeignet zur Behandlung von Erkrankungen, bei denen eine Hemmung der Gallensäurerückresorption im Darm, insbesondere im Dünndarm, wünschenswert erscheint, angesehen werden. Beispielsweise werden die chologene Diarrhoe nach Ileumresektion oder auch erhöhte Cholesterin-Blutspiegel auf diese Weise behandelt.

Für die als Lipidsenker in der Verwendung befindlichen Ionenaustauscherharze, wie Colestipol und Colestyramin, ist insbesondere eine sehr hohe Tagesdosis einzuhalten. Sie beträgt z.B. für Colestyramin 12 - 24 g, im Höchstfall 32 g, für Colestipol 15 - 30 g.

Diese hohe Dosierung sowie unangenehmer Geruch, Geschmack und Konsistenz erschweren die Patienten-Compliance.

Nebenwirkungen dieser Ionenaustauscherharze sind auch auf die mangelnde Selektivität (z.B. Avitaminosen) zurückzuführen. Für beide Präparate wurde eine therapeutische Bedeutung in Kombination mit anderen hypolipidämisch wirkenden Pharmaka wie Fibrate, HMG-CoA-Reduktase-Inhibitoren, Probucol (vgl. z.B. M.N. Cayen, Pharmac. Ther. 29, 187 (1985) und 8th International Symposium on Atherosclerosis, Rome, Oct. 9-13, 1988, Abstracts S. 544, 608, 710) beschrieben, wobei die erzielten Effekte auch die Therapie von schweren Hyperlipidämien ermöglichen. Deshalb erscheint es bedeutungsvoll, bei dem gegebenen Wirkprinzip geeignete Stoffe ohne die Nachteile der gegenwärtig verwendeten Präparate zu finden.

Folgende Merkmale der genannten Präparate und insbesondere von Colestipol sind als verbesserungswürdig anzusehen:
1. Die hohen Tagesdosen, die auf eine geringe Bindungsrate in isotonischer Lösung und auf teilweise Wiederfreisetzung der adsorbierten Gallensäure zurückzuführen sind.
2. Die qualitative Verschiebung der Gallensäurezusammensetzung der Galle mit abnehmender Tendenz für Chenodesoxycholsäure und die damit verbundene zunehmende Gefahr der Cholelithiasis.
3. Das Fehlen einer dämpfenden Wirkung auf den Cholesterinstoffwechsel der Darmbakterien.
4. Die zu hohe Bindungsrate von Vitaminen und Pharmaka, die Substitutionsbedarf an diesen Stoffen und Butspiegelkontrollen eventuell erforderlich macht.
5. Nicht ausreichende Reinheit und Stabilität der Polymere (bei Colestyramin Gefahr der Abspaltung von Amino- und Ammonium-Gruppen).
6. Unzureichende Patienten-Compliance aufgrund a) der "sandigen" Konsistenz (Colestyramin = Hardgel-Polymer) und b) des unangenehmen Geruchs und Geschmacks.

Variationen zu den bisher verwendeten Präparaten wie z.B. Einführung von Spacern zwischen Ammonium-Gruppen und Polymerhauptkette im Fall des Colestyramins (EP-A-0 404 062) führen zu keiner entscheidenden Verringerung der beschriebenen Nachteile.

Aufgabe der vorliegenden Erfindung war es, Verbindungen mit neuen Polymerstrukturen bereitzustellen, die Gallensäuren konzentrationsabhängig in hohem Maße binden. Darüberhinaus sollten diese Verbindungen die bestehenden Nachteile der bisher verwendeten Austauscherharze nicht bzw. nicht mehr im bekannten Maße aufweisen.

Mit den unlöslichen, wasserbindenden, stickstoffhaltigen Vinylcopolymeren der Formel I gelingt die Lösung der Aufgabe sowie eine Überwindung der beschriebenen Mängel.

Die Erfindung betrifft daher vernetzte, stickstoffhaltige Vinylcopolymere mit Einheiten der Formel I 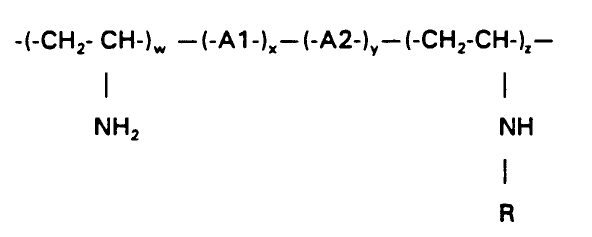 und deren physiologisch verträgliche Salze, wobei in Formel I
- w 0,1: -0,98,
- x 0,0: -0,8,
- y 0,0: - 0,3 und
- z 0,0: - 0,6 ist, wobei w + x + y + z gleich 1 ist, x = 0 nur falls y ≠ 0, y = 0 nur falls gleichzeitig x ≠ 0 und z ≠ 0 und R = R₄ sind,
und worin
- A1: 1. für einen hydrolysestabilen Rest der Formel 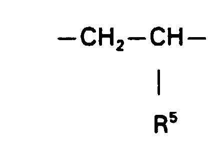 steht, worin R⁵ ein linearer oder verzweigter Alkoxyrest mit 3 bis 18 C-Atomen, ein Cycloalkylrest mit 5 bis 8 C-Atomen, ein Phenoxy-, Tolyl-, 4-(tert. Butyl)-phenyl-, 4-Aminophenyl-, N-Carbazyl-, N-lmidazolyl-, 2-Pyridinyl- oder 4-Pyridinylrest ist, oder
2. für einen hydrolysestabilen Rest der Formel 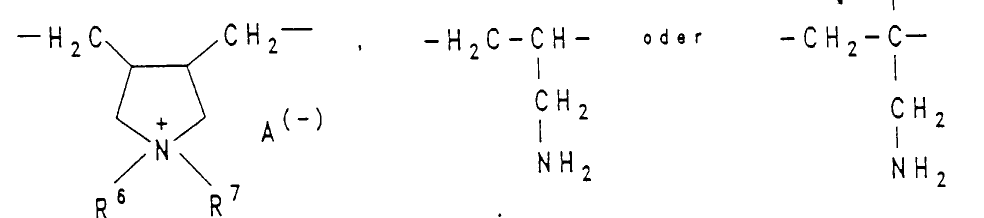 worin R⁶ ein Alkylrest mit 1 bis 18 C-Atomen ist, R⁷ H oder, falls R⁶ Alkyl mit 1 bis 6 C-Atome ist, ein Alkylrest mit 1 bis 6 C-Atomen ist,
   steht
- A2: 1. für den Rest eines hydrolysestabilen Vernetzers der Formel 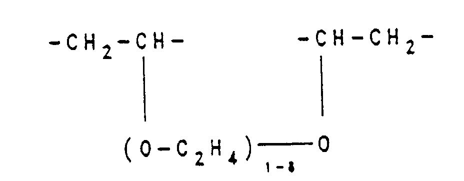 steht, oder
2. für einen Rest der Formel 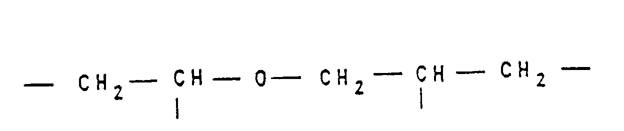 oder 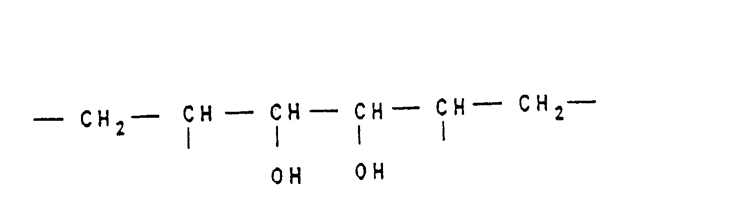 und worin
- R: für R¹-X, R²-X, R³ oder R⁴ steht, wobei
- R¹: einen Substitutenten bedeutet, ausgewählt aus der Gruppe bestehend aus:
1. -(CH₂)ₙ-CH₃, wobei n eine ganze Zahl von 3 bis 21 ist, verzweigtes Alkyl mit 3 bis 21 Kohlenstoffatomen, geradkettiges oder verzweigtes Alkenyl mit bis zu 21 Kohlenstoffatomen,
2. Cycloalkyl, Cycloalkenyl mit jeweils 5-12 Kohlenstoffatomen, ein-, zwei- oder dreifach substituiertes Cycloalkyl oder Cycloalkenyl mit jeweils 5-12 Ringkohlenstoffatomen und
3. Aryl, Arylalkyl, Arylalkenyl, wobei die Arylreste ein- oder mehrkernig sind, ein- bis dreifach substituiert sein können und Heteroatome enthalten können und
- X: eine Einfachbindung oder
eine Brückengruppe oder ein Spacer ist,
und
- R²: einen hydrophilen bzw. amphiphilen Substituenten bedeutet ausgewählt aus der Gruppe bestehend aus:
1. 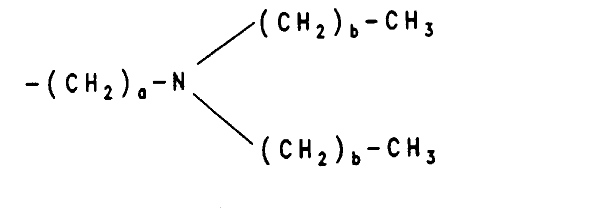
2. 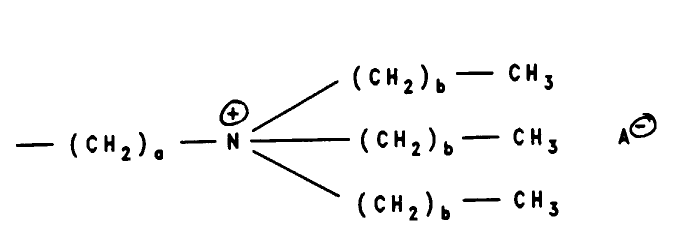
3. 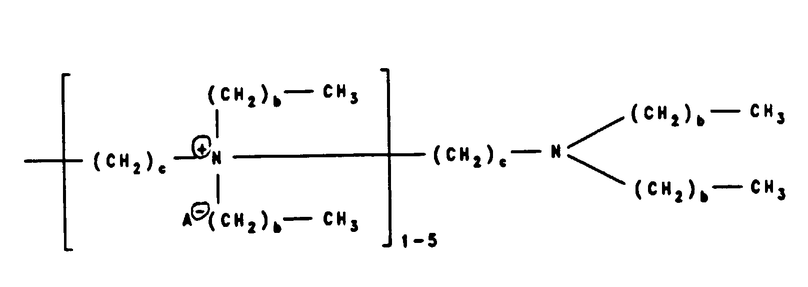
4. -(CH₂)_{c}-B, wobei B einen über N gebundenen Pyrrolidinyl-, Piperidinyl- oder Morpholinyl-Rest bedeutet,
5. -(CH₂)ₐ-D^{⊕} A^{⊖}, wobei D^{⊕} Pyridinium, Pyrimidinium oder Imidazolinium bedeutet, und
6. 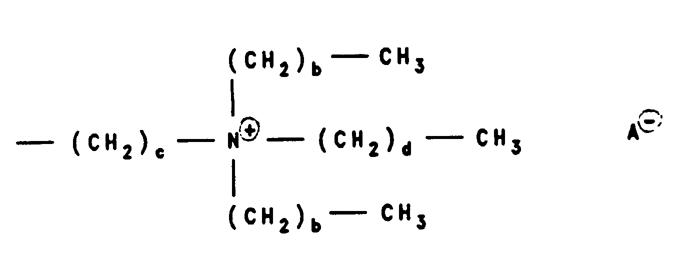 wobei bei den unter 1. bis 6. angegebenen Substituenten
   a eine ganze Zahl von 2 bis 16,
   b null, 1, 2 oder 3,
   c eine ganze Zahl von 2 bis 6,
   d eine ganze Zahl von 6 bis 17 und
   A ein physiologisch verträgliches Anion bedeuten,
- R³: für einen Rest steht ausgewählt aus der Gruppe bestehend aus
1. einer Cholsäure, die über die 3-α-OH- oder 24-COOH-Funktion direkt oder über einen Spacer gebunden ist,
2. einer Tauro- oder Glycocholsäure, die über die 3α-OH- oder Tauro- bzw. Glyco-Funktion direkt oder über einen Spacer gebunden ist, und
3. einen hydrophilen cyclischen Rest oder einen Glucopyranuronsäure-Rest, und
- R⁴: für einen vernetzenden Rest steht ausgewählt aus der Gruppe bestehend aus:
1. W-(CH₂)ₑ,W und
2. W-(CH₂-CH₂O)_{f}-CH₂-CH₂-W wobei W eine Einfachbindung, 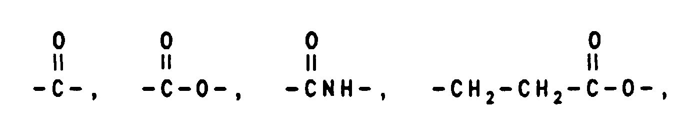 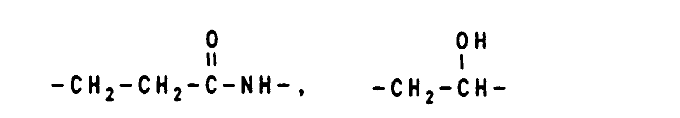 ist, und wobei
   e eine ganze Zahl von 3 bis 12,
   f eine ganze Zahl von 1 bis 6
   bedeutet.

In den vorstehenden und folgenden Ausführungen steht:
Aryl für einen ein- oder mehrkernigen aromatischen Kohlenwasserstoffrest mit 6 bis 14 C-Atomen, wobei im Falle der mehrkernigen Reste die Arylgruppen miteinander kondensiert, über C-C-Bindungen oder über Brückenglieder wie -O-, -COO- oder -CONH- miteinander verbunden sind.
Ferner umfaßt der Begriff Aryl auch 5- bis 14-gliedriges Heteroaryl mit 1 Heteroatom oder 2 nicht benachbarten, gleichen oder verschiedenen Heteroatomen, ausgewählt aus der Gruppe bestehend aus Sauerstoff und Stickstoff.

Aryl steht insbesondere für Phenyl, Arylalkyl für Benzyl und Phenylethyl und Arylalkenyl für

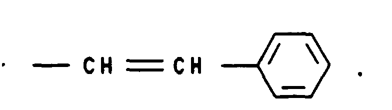

Beispiele für aromatische Reste mit 1 oder 2 Heteroatomen sind Reste der Chinolincarbon-, Benzimidazolcarbon-, Furancarbon-, Nicotin- und Cumarilsäure.

Die Cycloalkyl bzw. Cycloalkenylreste sind gegebenenfalls ein, zwei oder dreifach substituiert mit Hydroxy, (C₁-C₆)-Alkyl und/oder (C₁ -C₆)-Alkoxy-Resten, wobei im Falle der Mehrfachsubstitution die Substituenten gleich oder verschieden sind. Entsprechendes gilt auch für die Substituenten am Aryl; als Rest kommt z.B. ein Triethylbenzoesäurerest in Frage.

Das Brückenglied X bedeutet

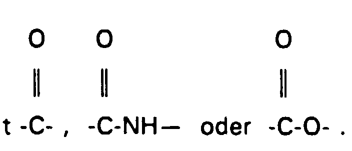

Der Spacer X besitzt die Formel 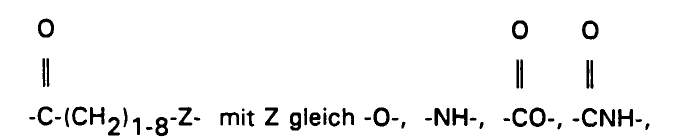 wobei im Fall von 3-8 Methylengruppen eine mittlere CH₂-Gruppe durch Sauerstoff ersetzt sein kann.

R¹-X ist z.B. ein Rest der Formel

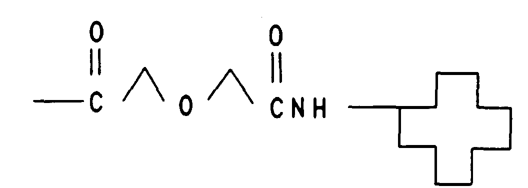

Ein Beispiel für R in der unter R³ 1. erwähnten Bedeutung ist der Rest der Formel 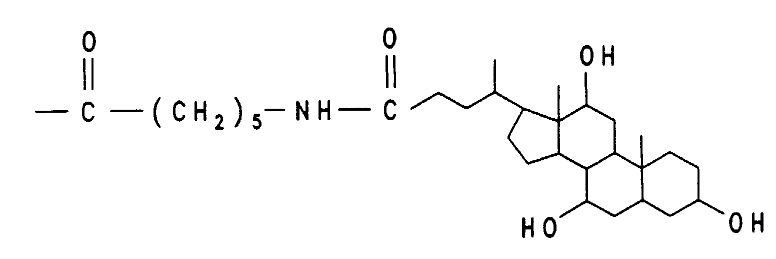 Ein hydrophiler cyclischer Rest R gemäß R³ 3. ist ein Cyclodextrinrest oder ein funktionalisierter 7 - 18gliedriger C-haltiger Azamakrozyklus mit 2 bis 4 Stickstoffatomen, und gegebenenfalls 2, 3 oder 4 Sauerstoffatomen, die durch Ethylengruppen getrennt sind, wie z.B. 1,4,7-Triazacyclononan, ein Cyclen- oder Cyclamrest oder 1,4-Diaza-18 crown 6.
Der Rest R¹ ist vorzugweise hydrophob.

Sofern b in einer Struktur mehrfach vorkommt, so ist b gleich oder verschieden, c ist in einer Struktur immer gleich, W ist immer gleich.

Die erfindungsgemäßen Vinylcopolymere mit Einheiten der Formel I sind verzweigt und vernetzt. Wie in der Polymerchemie üblich sind die in Formel I angegebenen Gruppen über das Gesamtpolymer statistisch verteilt bzw. gemäß ihrer Copolymerisationsparameter angeordnet.

Die Erfindung betrifft ferner ein Verfahren zur Herstellung von Vinylcopolymeren mit Einheiten der Formel I, welches dadurch gekennzeichnet ist, daß man aus Vinylformamid durch Copolymerisation eine Verbindung der Formel II 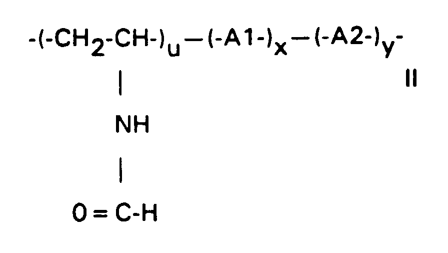 herstellt, wobei A1, A2, x und y die zu Formel I angegebenen Bedeutungen haben und u 0.1 - 0.98 ist, diese zu einer Verbindung der Formel III 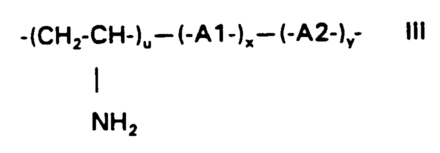 worin A1, A2, x und y die zu Formel I und u die zu Formel II angegebenen Bedeutungen haben, hydrolysiert, und anschließend gegebenenfalls nach üblichen Methoden derivatisiert, zu einer Verbindung mit Einheiten der Formel I oder deren physiologisch verträglichen Salzen.

Die vernetzten, stickstoffhaltige Copolymere werden hergestellt durch vernetzende Copolymerisation mit dem Monomeranteil A₂ oder durch polymeranaloge Vernetzung (Anteil R⁴) unter Verwendung von Vernetzern aus der Gruppe der Dialkylantien wie α,ω-Dibromalkane, Ditosylate, Diisocyanate oder aus der Gruppe der Disäuren, der Diacrylate und Diacrylamide oder der Diepoxide.

In Formel II steht u für die Summe aus w und z. In den erfindungsgemäßen Verbindungen liegen z Anteile als Derivat (R) und w Anteile als freie Vinylamineinheiten bzw. als deren physiologisch verträglich Salze vor. Die Derivatisierung der Vinylaminanteile u wird vorzugsweise so geführt, daß z kleiner als 0,6 mal w ist.
Bevorzugt sind Vinylcopolymere, bei denen R die Bedeutung R¹-X, R²-X oder R³ hat und bei denen gleichzeitig z 0,1 bis 0,3 ist. Falls R gleich R⁴ ist, ist z vorzugsweise 0,0 bis 0,1.
Bei der Herstellung der Copolymerisate beträgt der Anteil an Gruppen A1 0 bis 80 %, vorzugsweise 0 bis 50 %, insbesondere 10 bis 30 %. Der Anteil an Gruppen A2 beträgt 0 bis 30 %, vorzugsweise 2 bis 20 %, insbesondere 5 - 15 %.

Das nachfolgende Reaktionsschema erläutert das Verfahren am Beispiel einer vernetzenden Copolymerisation, z. B. mit y mal Triethylenglykoldivinylether

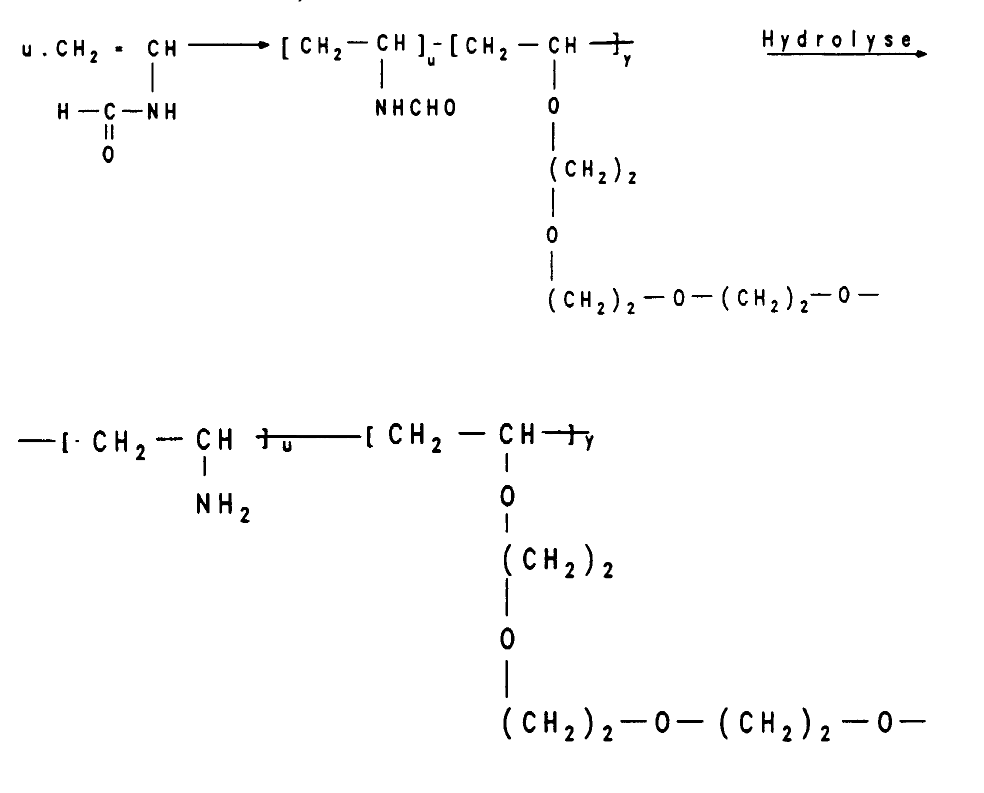

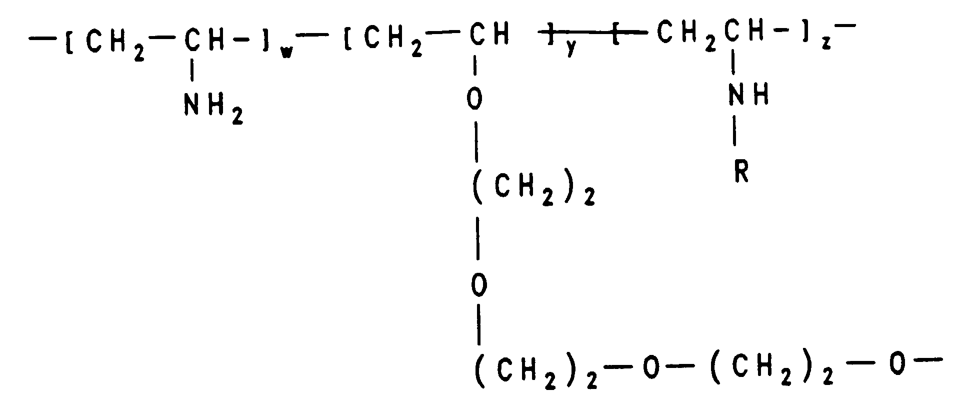

Aufgrund ihrer Eigenschaften können die Copolymere mit Einheiten der Formel I als Arzneimittel z.B. als Lipidsenker, als Sättigungsförderer, als Nahrungsmittelzusatz und Nahrungsmittelhilfsstoff verwendet werden. Die Erfindung betrifft daher auch diese Verwendungen und Arzneimittel auf Basis der Verbindungen mit Einheiten der Formel I.

Durch den Einsatz der erfindungsgemäßen Verbindungen kann die übliche Dosierung der bisher verwendeten Gallensäureadsorber zur Behandlung der Hypercholesterinämie erheblich gesenkt werden. Dadurch stellt sich das Problem der Dosierung und der Compliance nicht mehr. Zusätzlich wird die Compliance auch dadurch verbessert, daß die Verbindungen Softgel-Charakter besitzen, geschmacks- und geruchsneutral sind, so daß keine Geschmacks- und Geruchskompensatoren benötigt werden.

Die Effektivität der beschriebenen Wirkstoffe läßt sich durch spezielle Mikroformulierungen erhöhen. Hierzu werden die Verbindungen mittels verschiedener Techniken in quellbare Mikropartikel überführt (z.B. mechanische Mikronisierung). Mikropartikel lassen sich auch während der Synthese durch die Verfahrensführung, wie Latexpolymerisation, erhalten. Die quellbaren Mikropartikel zeichnen sich dadurch aus, daß der Wirkstoff auf einer sehr großen, adsorptiven Oberfläche verteilt ist, so daß die Gallensäure-Bindungsstellen gut zugänglich sind.

Als Darreichungsform können aus den Verbindungen Filmtabletten hergestellt werden, die in vitro die gleiche Wirksamkeit aufweisen wie die Verbindungen in Pulverform. Pro 250 mg Wirkstoff enthalten sie z.B. nur 40 mg pharmazeutisch übliche Hilfsmittel.

Die mit den Verbindungen zu erzielende Senkung des Serumcholesterinspiegels kann weiter verbessert werden durch gleichzeitige Verwendung von weiteren nicht systemisch wirkenden oder systemisch wirkenden Lipidsenkern (z.B. HMG-CoA-Reduktase-Inhibitoren) im Rahmen einer Kombinationstherapie.

Da die Verbindungen den enterohepatischen Kreislauf unterbrechen, eignen sie sich auch zum Einsatz bei oralen Vergiftungen.

Darüberhinaus können die Verbindungen aufgrund ihres Wasseraufnahmevermögens als Sättigungsförderer eingesetzt werden.

Da die erfindungsgemäßen Verbindungen gut quellbar sind und Säuren binden, können sie als Antacida zur Behandlung einer übermäßen Magensäureproduktion eingesetzt werden und somit als Mittel gegen Gastritis und Ulcus ventriculi oder duodeni Verwendung finden.

Aufgrund ihrer Wechselwirkung mit Cholesterin sind die Verbindungen in der Lage, das mit der Nahrung aufgenommene Cholesterin zu adsorbieren. Der Anteil des Cholesterins der Nahrung wird damit gebunden und nicht vom Körper resorbiert.

Weiterhin eignen sich die erfindungsgemäßen Verbindungen auch als Nahrungsmittelhilfsstoffe. So wird beispielsweise aus Milch oder Eibestandteilen Cholesterin adsorbiert. Die daraus resultierende Nahrungsmittel zeichnen sich durch reduzierten Cholesterinanteil aus.

Verbindungen mit Einheiten der Formel I sind als mucoadhäsive Transportsysteme für Wirkstoffe geeignet. Sie bilden stark hydratisierbare Polymermatrices, die Wasserstoffbrücken-bildende sowie kationische Gruppen besitzen, eine hohe Flexibilität der Polymerkette aufweisen und zusätzlich mit hydrophoben Einheiten substituiert sein können. Daher sind die Verbindungen in der Lage, die Verweildauer eines gebundenen oder adsorbierten Wirkstoffs im Magen oder Dünndarm zu verlängern. Sie werden als Wirkstoffträger an die Schleimschicht der Magen-Darm-Wand adsorbiert, wobei die positiv geladenen Gruppen der Copolymere mit den negativ geladenen Gruppen der endständigen Sialinsäuren der Mucinmoleküle in Wechselwirkung treten, um so einen verzögerten Transport der Wirkstoffe durch den Magen-Darm-Trakt zu bewirken. Gleichzeitig wird durch die Art der Wechselwirkung die Wirkstoffresorption verbessert.

### In vitro Tests

In einem in vitro Test werden die Verbindungen auf ihre Gallensäure-Bindungs-Kapazität geprüft.
10 mg Polymerprobe werden in 18 ml Puffer (PBS, pH 7,4, 15 mM oder 37 mM) gelöst und 1 Stunde bei Raumtemperatur gerührt, mit 2 ml Na-Taurocholat (37 mM) versetzt und 2 Stunden bei Raumtemperatur inkubiert. Die festen Polymer-Taurocholat-Komplexe werden durch Filtration abgetrennt. Die Bestimmung des Anteils an nicht gebundenem Taurocholat erfolgt mittels HPLC (Säule: RP-8® LiChrosorb; Eluent: Methanol/Citratpuffer pH 4 (Riedel de Haen)/Wasser 6:1:1).

Bei einem Mischungsverhältnis 1/4 (w/w) von Polymerprobe zu Natriumtaurocholat ergeben sich die folgenden Taurocholat-Adsorptionwerte im molaren Verhältnis:
Verbindung Beispiel 1) : 17 mol %
Verbindung Beispiel 2) : 25 mol %
Verbindung Beispiel 3) : 24 mol %
Verbindung Beispiel 5): 26 mol %
Verbindung Beispiel 6): 29 mol %
Verbindung Beispiel 7): 25 mol %

### Beispiele:

### Beispiel 1

120 g Vinylformamid und 52 g Triethylenglykoldivinylether werden mit 1,4 g 4,4'-Azocyanopentansäure in Toluol gelost und mit N₂ gespült. Es wird 30 min bei 60 °C und 6 h bei 70 °C gerührt. Das ausgefallene Polymer wird abgesaugt, mit Toluol gewaschen und getrocknet. Ausbeute: 161 g
152 g des Copolymers werden in 2.5 l halbkonz. HCl suspendiert und 2 h bei 80 °C gerührt. Nach Zugabe von 0.5 l konz. HCl wird weitere 6 h bei 80 °C gerührt. Das Produkt wird abgesaugt, zur Einstellung des Hydrochloridgehalts in H₂O resuspendiert und auf pH = 4 titriert. Es wird erneut in Aceton gefällt und mehrfach mit Aceton ausgerührt, bevor es bei 50 °C im Vakuum getrocknet wird.
Ausbeute: 117 g

### Beispiel 2

60 g Vinylformamid, 8,6 g Triethylenglykoldivinylether und 0,6 g ACPS werden wie unter 1) beschrieben in 350 ml Toluol polymerisiert und anschließend hydrolysiert. Zur Hydrochloridgehalt-Einstellung wird in Ethanol/H₂O 1:1 resuspendiert.

### Beispiel 3

3 g Verbindung aus Beispiel 1 werden in 200 ml H₂O bei pH 10 dispergiert und mit 0,55 g Dimethylchlorethylamin-Hydrochlorid versetzt und bei gleichem pH-Wert 8 h bei 80 °C gerührt. Das Polymer wird in Aceton gefällt, in H₂O/Ethanol aufgenommen, auf pH = 4 titriert und erneut in Aceton gefällt und ausgerührt. Die Trocknung erfolgt bei 50 °C im Vakuum.

### Beispiel 4

5 g Vinylformamid, 1,3 g Vinylimidazol, 1,4 g Triethylenglykoldivinylether und 0,07 g Azocyanopentansäure werden in 40 ml Wasser gegeben. Die Lösung wird mit N₂ gespült und 6 h bei 60 °C gerührt. Nach Zugabe von 40 ml H₂O und 60 ml halbkonzentrierter HCl wird 3 h bei 70 °C gerührt und nach Zugabe von 90 ml konz. HCl weiter 4h bei 70 °C. Der Ansatz wird mit Aceton versetzt, das Produkt abgesaugt, in H₂O suspendiert und mit NaOH auf pH 4 eingestellt. Es wird erneut in Aceton ausgefällt und mehrfach mit Aceton ausgerührt bevor es bei 50 °C im Vakuum getrocknet wird.

### Beispiel 5

5,7 g Vinylformamid, 1,9 g N-Vinylimidazol und 0,1 g Azocyanopentansäure werden in 47 ml H₂O gegeben, mit N₂ gespült und 7h bei 70°C gerührt. Das entstandene Copolymer wird in Aceton gefällt, zur Hydrolyse in H₂O gelöst und mit 2 Mol NaOH pro Mol Formamid-Anteil 6h bei 70°C gerührt. Der Ansatz wird mit HCl auf pH 6 eingestellt, mit 1 Teil Methanol verdünnt und in Aceton gefällt. Substitutionsgrad laut NMR: 20 %.
Ausbeute: 5.0 g
2 g des Copolymers werden in 15 ml H₂O mit NaOH auf pH 10 eingestellt, mit 0,36 g Dibromhexan (5 mol-%) und 120 mg NaOH versetzt und 6h bei 90°C gerührt. Nach 1/2 h setzt die Gelbildung ein.
Mit 1n HCl wird der pH-Wert auf 1 gestellt und durch Zugabe von Aceton invers gefällt. Bei pH 5 erfolgen 2 weitere Fällungen in Aceton.
Ausbeute: 2,1 g

### Beispiel 6

Analog zu Beispiel 5 werden 5 g Vinylformamid und 2,8 g N-Vinylimidazol mit 0,11 g Azocyanopentansäure in 50 ml H₂O 7h bei 70°C polymerisiert. Anschließend erfolgt die basische Hydrolyse.
Substitutionsgrad laut NMR: 32 %
Ausbeute: 4,4 g
1,5 g des Copolymers werden mit 0,26 g Dibromhexan und 84 mg NaOH in 15 ml H₂O vernetzt. Die Aufarbeitung erfolgt analog Beispiel 5.
Ausbeute: 1,2 g.

### Beispiel 7

Analog zu Beispiel 5 werden 3,6 g Vinylformamid und 4,7 g N-Vinylimidazol mit 0,12 g Azocyanopentansäure in 52 ml H₂O 7h bei 70°C polymerisiert. Die basische Hydrolyse erfolgt mit 1,4 g NaOH.
Substitutionsgrad laut NMR: 53 %.
Ausbeute: 4,8 g.
1,5 g des Copolymers werden mit 0,24 g Dibromhexan und 77 mg NaOH in 15 ml H₂O vernetzt. Die Aufarbeitung erfolgt analog Beispiel 5.
Ausbeute: 0,9 g.

## Patentansprüche

1. Vernetzte, stickstoffhaltige Vinylcopolymere mit Einheiten der Formel I 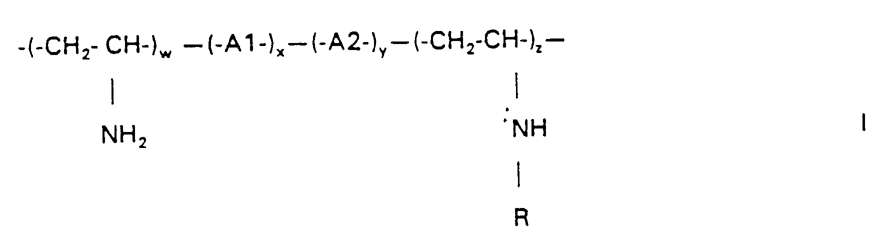 und deren physiologisch verträgliche Salze, wobei in Formel I
w 0,1 - 0,98,
x 0,0 - 0,8,
y 0,0 - 0,3 und
z 0,0 - 0,6 ist, wobei w + x + y + z gleich 1 ist, x = 0 nur falls y ≠ 0, y = 0 nur falls gleichzeitig x ≠ 0 und z ≠ 0 und R = R⁴ sind,
und worin
A1
1. für einen hydrolysestabilen Rest der Formel 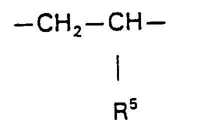 steht, worin R⁵ ein linearer oder verzweigter Alkoxyrest mit 3 bis 18 C-Atomen, ein Cycloalkylrest mit 5 bis 8 C-Atomen, ein Phenoxy-, Tolyl-, 4-(tert. Butyl)-phenyl-, 4-Aminophenyl-, N-Carbazyl-, N-Imidazolyl-, 2-Pyridinyl- oder 4-Pyridinylrest ist, oder
2. für einen hydrolysestabilen Rest der Formel 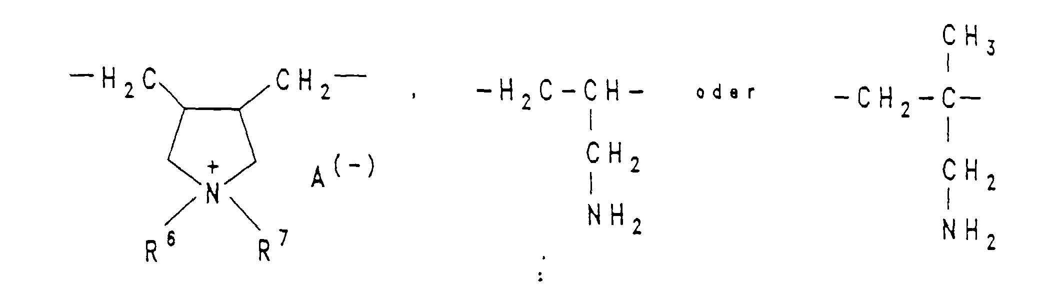 worin R⁶ ein Alkylrest mit 1 bis 18 C-Atomen ist, R⁷ H oder, falls R⁶ Alkyl mit 1 bis 6 C-Atome ist, ein Alkylrest mit 1 bis 6 C-Atomen ist,
steht
A2
1. für den Rest eines hydrolysestabilen Vernetzers der Formel 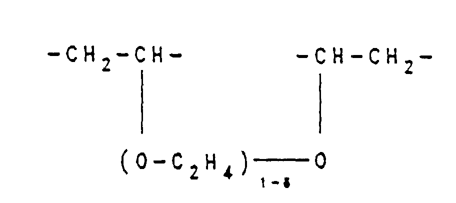 steht, oder
2. für einen Rest der Formel 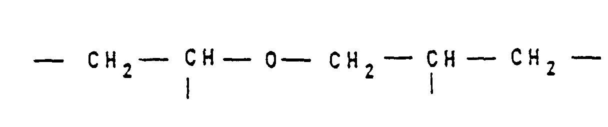 oder 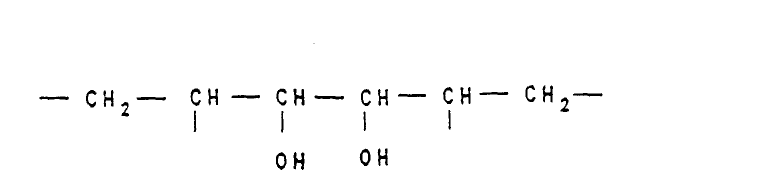 und worin
R für R¹-X, R²-X, R³ oder R⁴ steht, wobei
R¹ einen Substitutenten bedeutet, ausgewählt aus der Gruppe bestehend aus:
1. -(CH₂)ₙ-CH₃, wobei n eine ganze Zahl von 3 bis 21 ist, verzweigtes Alkyl mit 3 bis 21 Kohlenstoffatomen, geradkettiges oder verzweigtes Alkenyl mit bis zu 21 Kohlenstoffatomen,
2. Cycloalkyl, Cycloalkenyl mit jeweils 5-12 Kohlenstoffatomen, ein-, zwei- oder dreifach substituiertes Cycloalkyl oder Cycloalkenyl mit jeweils 5-12 Ringkohlenstoffatomen und
3. Aryl, Arylalkyl, Arylalkenyl, wobei die Arylreste ein- oder mehrkernig sind, ein- bis dreifach substituiert sein können und Heteroatome enthalten können und
X eine Einfachbindung oder
eine Brückengruppe oder ein Spacer ist,
und
R² einen hydrophilen bzw. amphiphilen Substituenten bedeutet ausgewählt aus der Gruppe bestehend aus:
1. 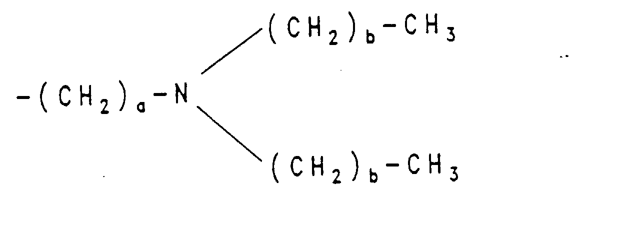
2. 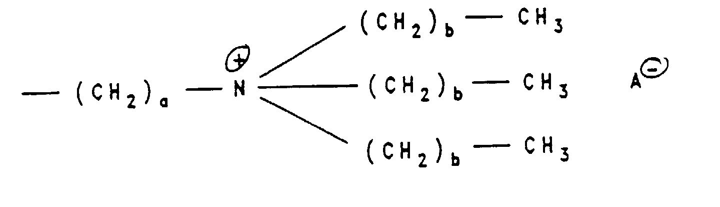
3. 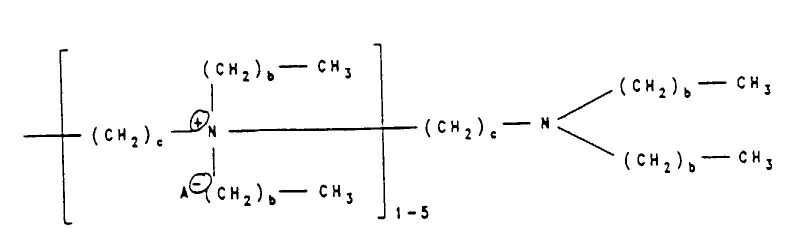
4. -(CH₂)_{c}-B, wobei B einen über N gebundenen Pyrrolidinyl-, Piperidinyl- oder Morpholinyl-Rest bedeutet,
5. -(CH₂)ₐ-D^{⊕} A^{⊖}, wobei D^{⊕} Pyridinium, Pyrimidinium oder Imidazolinium bedeutet,
6. 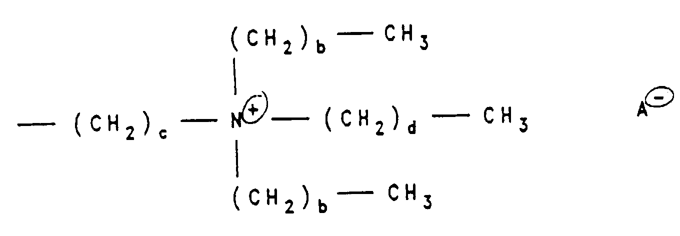 wobei bei den unter 1. bis 6. angegebenen Substituenten
a eine ganze Zahl von 2 bis 16,
b null, 1, 2 oder 3,
c eine ganze Zahl von 2 bis 6,
d eine ganze Zahl von 6 bis 17 und
A ein physiologisch verträgliches Anion bedeuten oder
R³ für einen Rest steht ausgewählt aus der Gruppe bestehend aus
1. einer Cholsäure, die über die 3-α-OH- oder 24 COOH-Funktion direkt oder über einen Spacer gebunden ist, oder
2. einer Tauro- oder Glycocholsäure, die über die 3α-OH- oder Tauro- bzw. Glyco-Funktion direkt oder über einen Spacer gebunden ist und
3. einen hydrophilen cyclischen Rest oder einen Glucopyranuronsäure-Rest,
R⁴ für einen vernetzenden Rest steht ausgewählt aus der Gruppe bestehend aus:
1. W-(CH₂)ₑ-W und
2. W-(CH₂-CH₂O)_{f}-CH₂-CH₂-W wobei W eine Einfachbindung, 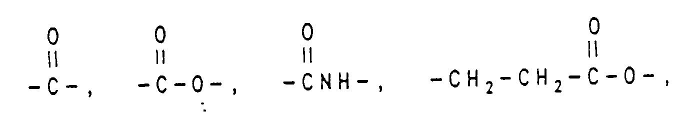 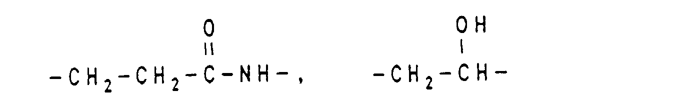 ist, und wobei
e eine ganze Zahl von 3 bis 12,
f eine ganze Zahl von 1 bis 6
bedeutet.

2. Verfahren zur Herstellung der Vinylcopolymere gemäß Anspruch 1, dadurch gekennzeichnet, daß man aus Vinylformamid durch Copolymerisation eine Verbindung der Formel II 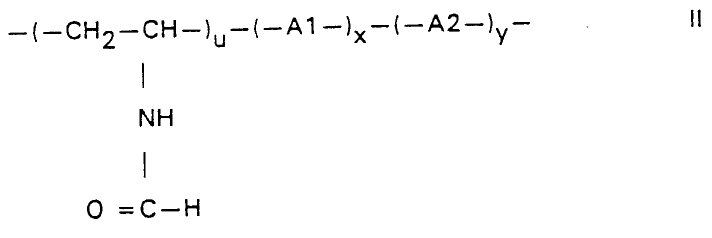 herstellt, wobei A1, A2, x und y die zu Formel I angegebenen Bedeutungen haben und u 0.1 - 0.98 (u steht für die Summe aus w und z) ist, diese zu einer Verbindung der Formel III 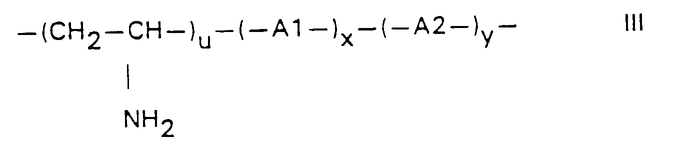 worin A1, A2, x und y die zu Formel I und u die zu Formel II angegebenen Bedeutungen haben, hydrolysiert, und anschließend gegebenenfalls nach üblichen Methoden derivatisiert, zu einer Verbindung der Formel I oder deren physiologisch verträglichen Salze.

3. Pharmazeutisches Präparat enthaltend eine Verbindung gemäß Anspruch 1.

4. Verwendung einer Verbindung gemäß Anspruch 1 als Arzneimittel.

5. Verwendung einer Verbindung gemäß Anspruch 1 als Hypolipidämikum.

6. Verwendung einer Verbindung gemäß Anspruch 1 als Sättigungsförderer, Nahrungsmittelzusatz oder Nahrungsmittelhilfsstoff.

7. Verwendung einer Verbindung gemäß Anspruch 1 zur Adsorption von Cholesterin.

8. Verwendung einer Verbindung gemäß Anspruch 1 als Antidot oder Antacida.

9. Verwendung einer Verbindung gemäß Anspruch 1 in Kombination mit systemisch oder nicht systemisch wirkenden Hypolipidämika.

10. Mikropartikel erhalten durch Gefriertrocknung oder Mikronisierung von Polyvinylaminen gemäß Anspruch 1.

11. Verwendung einer Verbindung gemäß Anspruch 1 als Wirkstoffträger.

## Claims

1. A crosslinked nitrogen-containing vinyl copolymer having units of the formula I 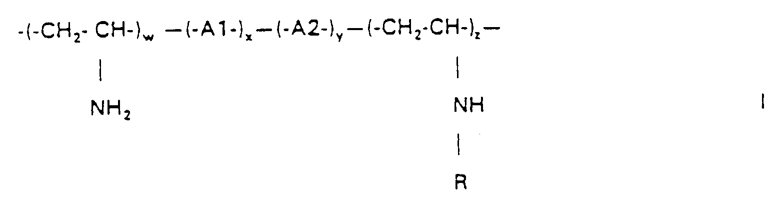 or a physiologically tolerated salt thereof, in which, in formula I,
w is 0.1 - 0.98,
x is 0.0 - 0.8,
y is 0.0 - 0.3 and
z is 0.0 - 0.6, in which w + x + y + z is 1, x = 0 only if y ≠ 0, y = 0 only if at the same time x ≠ 0 and z ≠ 0 and R = R⁴,
and in which
A1
1. is a hydrolysis-stable radical of the formula 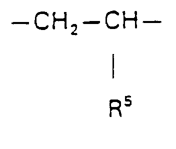 in which R⁵ is a linear or branched alkoxy radical having 3 to 18 carbon atoms a cycloalkyl radical having 5 to 8 carbon atoms or a phenoxy, tolyl, 4-(tert-butyl)-phenyl, 4-aminophenyl, N-carbazyl, N-imidazolyl, 2-pyridinyl or 4-pyridinyl radical, or
2. is a hydrolysis-stable radical of the formula 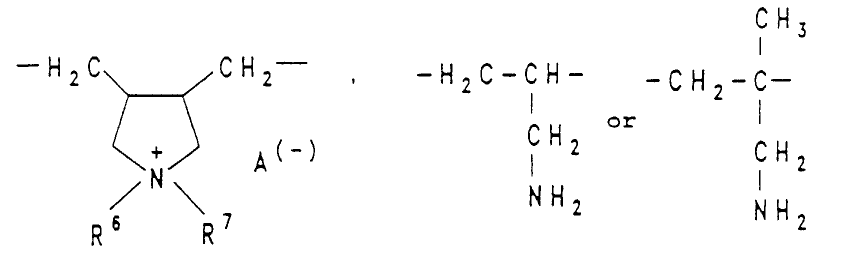 in which R⁶ is an alkyl radical having 1 to 18 carbon atoms and R⁷ is H or, if R⁶ is alkyl having 1 to 6 carbon atoms, an alkyl radical having 1 to 6 carbon atoms,
A2
1. is the radical of a hydrolysis-stable crosslinking agent of the formula 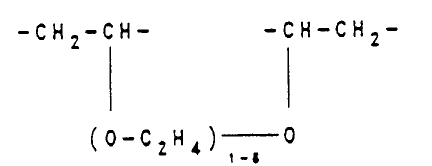 or
2. is a radical of the formula 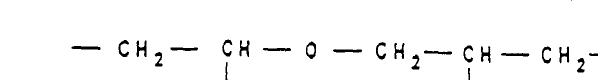 or 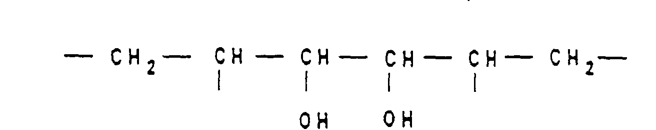 and in which
R is R¹-X, R²-X, R³ or R⁴, in which
R¹ is a substituent selected from the group comprising:
1. -(CH₂)ₙ-CH₃, in which n is an integer from 3 to 21, branched alkyl having 3 to 21 carbon atoms or straight-chain or branched alkenyl having up to 21 carbon atoms,
2. cycloalkyl, cycloalkenyl having in each case 5-12 carbon atoms or mono-, di- or trisubstituted cycloalkyl or cycloalkenyl having in each case 5-12 ring carbon atoms and
3. aryl, arylalkyl or arylalkenyl, in which the aryl radicals are mono- or polynuclear, can be mono- to trisubstituted and can contain hetero atoms and
X is a single bond or
a bridge group or a spacer,
and
R² is a hydrophilic or amphiphilic substituent selected from the group comprising:
1. 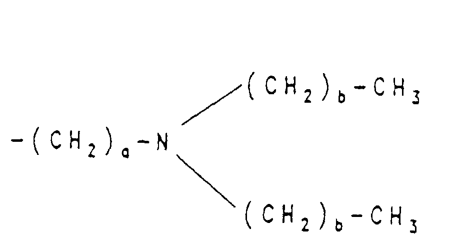
2. 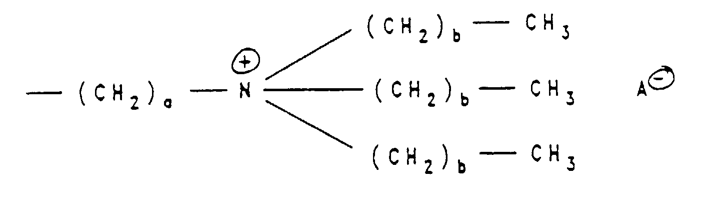
3. 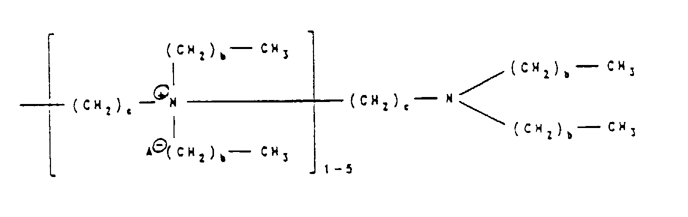
4. -(CH₂)_{c}-B, in which B is a pyrrolidinyl, piperidinyl or morpholinyl radical bonded via N,
5. -(CH₂)ₐ-D^{⊕}A^{⊖}, in which D^{⊕} is pyridinium, pyrimidinium or imidazolinium and
6. 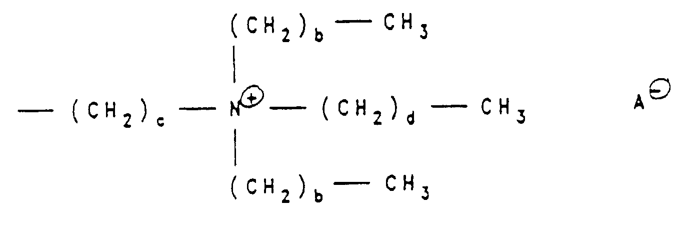 in which, in the substituents mentioned under 1. to 6.,
a is an integer from 2 to 16,
b is zero, 1, 2 or 3,
c is an integer from 2 to 6,
15 d is an integer from 6 to 17 and
A is a physiologically tolerated anion, or
R³ is a radical selected from the group comprising
1. a cholic acid which is bonded via the 3-α-OH or 24-COOH function directly or via a spacer, or
2. a tauro- or glycocholic acid which is bonded via the 3α-OH or tauro or glyco function directly or via a spacer and
3. a hydrophilic cyclic radical or a glucopyranuronic acid radical and
R⁴ is a crosslinking radical selected from the group comprising:
1. W-(CH₂)ₑ-W and
2. W-(CH₂-CH₂O)_{f}-CH₂-CH₂-W in which W is a single bond, 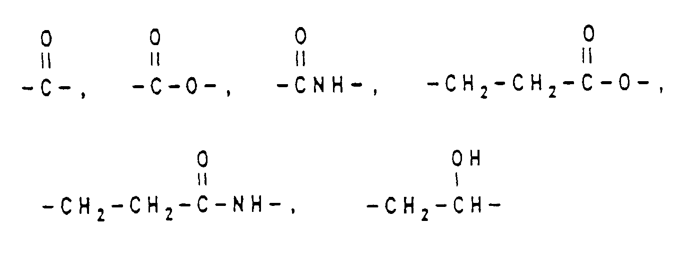 and in which
e is an integer from 3 to 12 and
f is an integer from 1 to 6.

2. A process for the preparation of a vinyl copolymer as claimed in claim 1, which comprises preparing a compound of the formula II 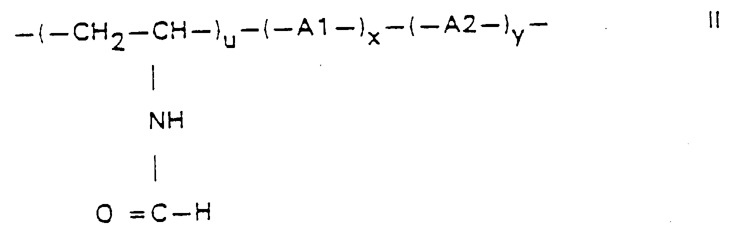 in which A1, A2, x and y have the meanings given in the case of formula I and u is 0.1-0.98 (u is the sum of w and z), from vinylformamide by copolymerization, hydrolyzing this product to give a compound of the formula III 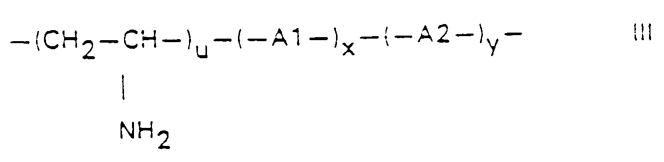 in which A1, A2, x and y have the meanings given in the case of formula I and u has the meaning given in the case of formula II, and then optionally forming derivatives by customary methods, to give a compound of the formula I or a physiologically tolerated salt thereof.

3. A pharmaceutical preparation comprising a compound as claimed in claim 1.

4. The use of a compound as claimed in claim 1 as a medicament.

5. The use of a compound as claimed in claim 1 as a hypolipidemic agent.

6. The use of a compound as claimed in claim 1 as a satiation promoter, foodstuff additive or foodstuff auxiliary.

7. The use of a compound as claimed in claim 1 for adsorption of cholesterol.

8. The use of a compound as claimed in claim 1 as an antidote or an antacid.

9. The use of a compound as claimed in claim 1 in combination with systemically or non-systemically acting hypolipidemic agents.

10. Microparticles obtained by freeze-drying or micronization of a polyvinylamine as claimed in claim 1.

11. The use of a compound as claimed in claim 1 as an active compound carrier.

## Revendications

1. Copolymères vinyliques réticulés, contenant de l'azote, ayant des motifs de Formule I 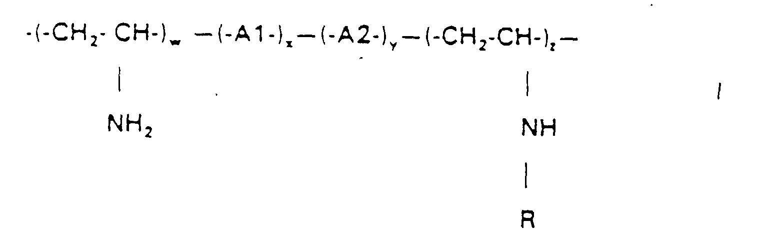 et leurs sels physiologiquement acceptables Formule I dans laquelle
w est compris entre 0,1 et 0,98
x est compris entre 0,0 et 0,8
y est compris entre 0,0 et 0,3,
z est compris entre 0,0 et 0,6, et dans laquelle w + x + y + z est égal à 1, x = 0 seulement lorsque y ≠ 0, y = 0 seulement lorsqu'en même temps x ≠ 0 et z ≠ 0 et R = R⁴,
et dans laquelle
A1
1. représente un résidu résistant à l'hydrolyse de formule 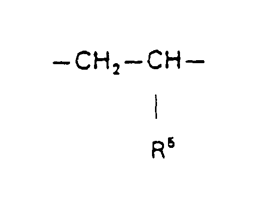 dans laquelle R est un groupe alcoxy linéaire ou ramifié avec de 3 à 18 atomes de carbone, un groupe cycloalkyle avec de 5 à 8 atomes de carbone, un groupe phénoxy, tolyle, 4-(tert.-butyl)-phényle, 4-aminophényle, N-carbazyle, N-imidazolyle, 2-pyridinyle ou 4-pyridinyle, ou
2. représente un radical résistant à l'hydrolyse de formule 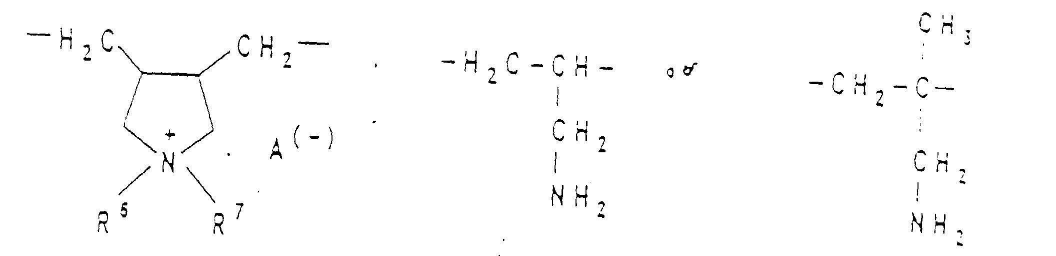 dans laquelle R⁶ est un groupe alkyle avec de 1 à 18 atomes de carbone, R⁷ est un atome d'hydrogène ou, si R⁶ est un groupe alkyle avec de 1 à 6 atomes de carbone, un radical alkyle avec de 1 à 6 atomes de carbone,
A2
1. représente le résidu d'un agent réticulant de formule 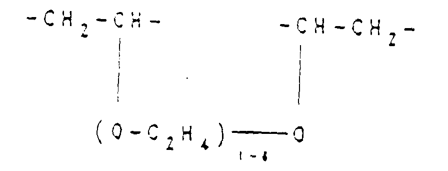 , ou
2. représente un résidu de formule 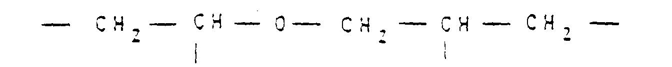 ou 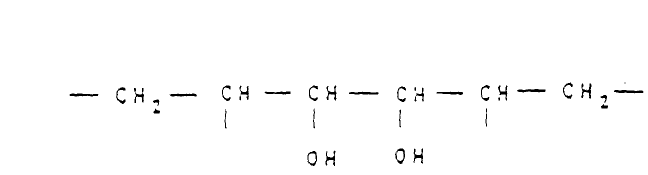 et dans laquelle
R représente R¹-X, R²-X, R³, ou R⁴, formules dans lesquelles
R¹ représente un substituant choisi dans le groupe constitué de :
1. -(CH₂)ₙ-CH₃, dans lequel n est un nombre entier compris entre 3 et 21, un groupe alkyle ramifié avec de 3 à 21 atomes de carbone, ou un groupe alkényle linéaire cu ramifié ayant jusqu'à 21 atomes de carbone ,
2. un groupe cycloalkyle, cycloalkényle avec respectivement 5-12 atomes de carbone, cycloalkyle ou cylcoalkényle mono, bi ou trisubstitué avec respectivement 5-12 atomes de carbone dans le cycle et
3. un groupe aryle, arylalkyle, arylalkényle dans lequel les radicaux aryle sont à un ou plusieurs noyaux, peuvent être mono, bi ou trisubstitués et peuvent contenir des hétéroatomes et
X représente une simple liaison ou est un groupe pontant ou un intercalaire, et
R² représente un substituant hydrophile ou amphiphile choisi dans le groupe constitué de :
1. 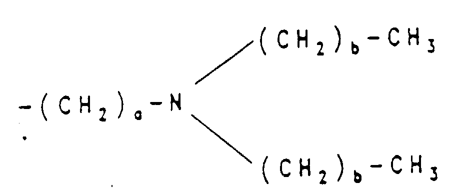
2. 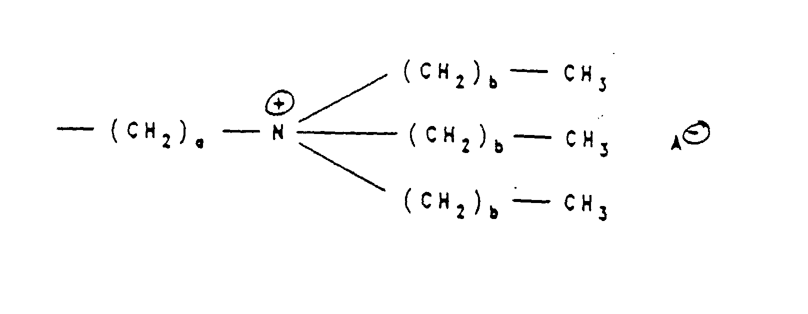
3. 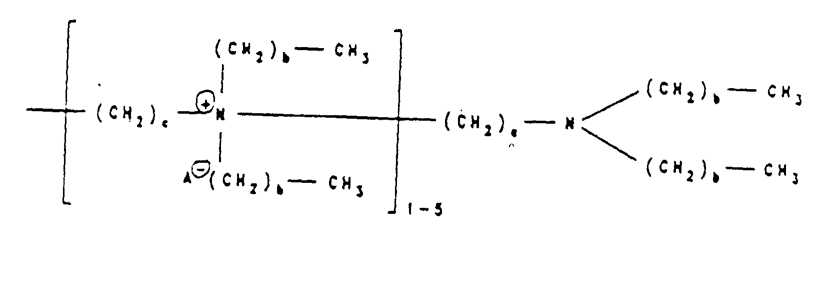
4. -(CH₂)_{c}-B, dans lequel B représente un radical pyrrolidinyle, pipéridinyle ou morpholinyle lié par l'azote,
5. -(CH₂)ₐ-D^{⊕}A^{⊖}, dans lequel -D^{⊕} représente le cation pyridinium, pyrimidinium ou imidazolinium, et
6. 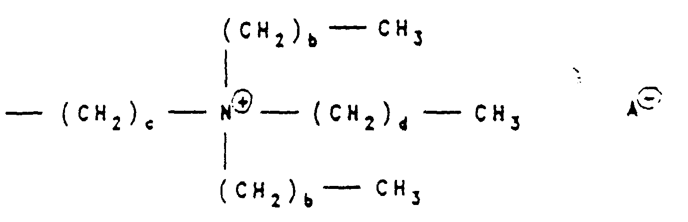 formules dans lesquelles pour les substituants donnés de 1. à 6.
a représente un nombre entier de 2 à 16,
b zéro, 1, 2 ou 3,
c un nombre entier de 2 à 6,
d un nombre entier de 6 à 17 et
A un anion physiologiquement acceptable,
R³ représente un résidu choisi dans le groupe constitué:
1. d'un acide cholique qui est lié directement ou par un intermédiaire sur la fonction OH en position 3-α ou COOH en position 24,
2. d'un acide taurocholique ou glycocholique qui est lié directement ou par un intermédiaire sur la fonction OH 3-α ou à la fonction tauro ou glyco et
3. d'un radical cyclique hydrophile ou d'un acide glycopyranuronique, et
R⁴ représente un radical réticulant choisi dans le groupe constitué :
1. W-(CH₂)ₑ-W
2. W-(CH₂-CH₂O)_{f}-CH₂-CH₂-W dans lequel W est une simple liaison, 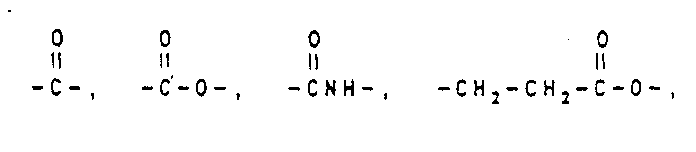 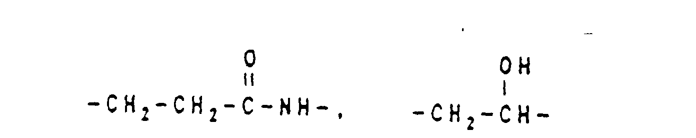 et dans lequel
e est un nombre entier de 3 à 12,
f est un nombre entier de 1 à 6.

2. Procédé de fabrication des copolymères vinyliques selon la revendication 1, caractérisé en ce que l'on fabrique par copolymérisation à partir de vinylformamide un composé de Formule II 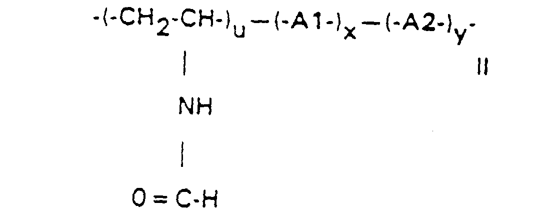 dans laquelle A1, A2, x et y ont la signification donnée pour le Formule I et u est compris entre 0,1 et 0,98 (u représente la somme de w et z), qu'on hydrolyse en un composé de Formule III 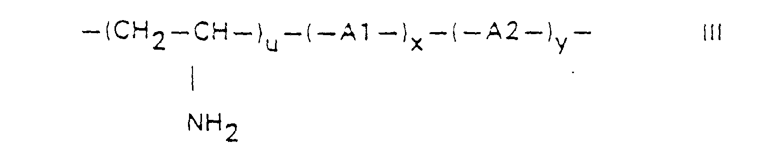 dans laquelle A1, A2, x et y ont la signification donnée pour la Formule I et u la signification donnée pour la Formula II , et qu'on transforme en dérivé ensuite éventuellement selon les procédés habituels pour obtenir un composé avec des motifs de Formule I ou de ses sels physiologiquement acceptables.

3. Préparation pharmaceutique contenant un composé selon la revendication 1.

4. Utilisation d'un composé selon la revendication 1 comme médicament.

5. Utilisation d'un composé selon la revendication 1 comme agent hypolipémiant.

6. Utilisation d'un composé selon la revendication 1 comme inducteur de satiété, comme additif alimentaire ou aliment auxiliaire.

7. Utilisation d'un composé selon la revendication 1 pour l'adsorption du cholestérol.

8. Utilisation d'un composé selon la revendication 1 comme antidote ou antiacides.

9. Utilisation d'un composé selon la revendication 1 en combinaison avec des agents hypolipémiants agissant de manière systémique ou non systémique.

10. Microparticules obtenues par lyophilisation ou micronisation de polyvinylamines selon la revendication 1.

11. Utilisation d'un composé selon la revendication 1 comme véhicule de principe actif.
